# EUROPÄISCHE PATENTSCHRIFT

(11) **EP 1 095 636 B1**
(45) Veröffentlichungstag und Bekanntmachung des Hinweises auf die Patenterteilung: **19.04.2006**
(21) Anmeldenummer: 00250344.9
(22) Anmeldetag: 19.10.2000
(51) Int. Cl.: A61L 31/08, A61F 2/06

(54) **Stent mit ungleichmässiger Beschichtung**
Stent having an uneven coating
Stent à revêtement irrégulier

(30) Priorität: 26.10.1999 DE 19951477
(43) Veröffentlichungstag der Anmeldung: 02.05.2001
(73) Patentinhaber: BIOTRONIK Mess- und Therapiegeräte GmbH & Co Ingenieurbüro Berlin, 12359 Berlin (DE)
(72) Erfinder: Schaldach, Max, 91054 Erlangen (DE); Kranz, Curt, 14163 Berlin (DE)
(74) Vertreter: Lindner-Vogt, Karin L.

(56) Entgegenhaltungen:
- EP-A- 0 689 807
- EP-A- 0 824 900
- EP-A- 0 916 317
- EP-A- 0 923 953
- DE-A- 19 913 978
- US-A- 5 674 276

## Beschreibung

Die Erfindung betrifft einen Stent mit einer Beschichtung.

Stents sind aus dem Stand der Technik in vielfältiger Weise bekannt. Stents werden u.a. im Zusammenhang mit der perkutanen transluminalen Angioplastie (PCTA, Percutaneous Transluminal Balloon Angioplasty) in der Gefäßchirurgie des Herzens verwendet. Stents können jedoch auch dazu dienen, andere Körperöffnungen aufzuweiten oder aufgeweitet zu halten. Diesem medizinischen Verfahren geht zunächst die Bestimmung des Ortes der Verengung eines Herzkranzgefäßes voraus. Anschließend wird ein sogenannter Angioplastieballon in der Arterie, die die Verengung, die sogenannte Stenose aufweist, an den Ort der Stenose gebracht und dort aufgeblasen. Durch die radial nach außen gerichtete Kraft des aufgeblasenen Ballons wird die Verengung aufgeweitet und im optimalen Fall der ursprüngliche Durchtrittsquerschnitt der zuvor verengten Arterie wieder hergestellt.

Neben der erfolgreichen Aufweitung des Gefäßes kann es jedoch zu Nebenwirkungen kommen, die lokale Risse in der Arterie, Zersetzungen und Vorsprünge von Plättchen in das Lumen der Arterie umfassen, so daß es trotz der Aufweitung zu einer Blockade des Gefäßes kommen kann. Darüber hinaus ist es möglich, daß durch elastisches Zurückfedern der Gefäßwand und/oder durch das Wachstum der Intima des Gefäßes erneut eine Stenose auftreten kann. Dies tritt statistisch innerhalb von sechs Monaten bei über 30% der Patienten auf, die mit PCTA behandelt wurden.

Um nun unmittelbar nach der Aufweitung des Gefäßes eine relativ glatte Innenwand des Gefäßes sicherzustellen und eine erneute Stenose vermeiden zu können, wurden Stents entwickelt. Diese kleinen Röhrchen dienen u.a. im Zusammenhang mit der PCTA dazu, den durch die Ballonangioplastie erzeugten Gefäßdurchtrittsquerschnitt aufrecht zu erhalten, um somit einen langfristigen Erfolg der PCTA sicherzustellen.

Der Erfolg dieses sogenannten Stenting hängt u.a. auch davon ab, wie schnell sich Blutpartikel an den in das Gefäß eingesetzten Stent anlagern. Denn je schneller sich Blutpartikel an die Stentoberflächen anlagern, um so schneller kommt es im Bereich der Stents erneut zu Gefäßverengungen aufgrund dieser Anlagerungen. Es ist also wünschenswert, Stentoberflächen zur Verfügung zu stellen, die es den Blutpartikeln erschweren, sich an der Oberfläche der Stents anzulagern und somit den Durchtrittsquerschnitt des mit einem Stent versehenen Blutgefäßes zu verengen.

Um derartige Anlagerungen zu verhindern oder zumindest zu erschweren, ist es daher aus dem Stand der Technik bekannt geworden, die Oberflächen der Stents mit einem Material zu beschichten, welches eine Anlagerung von Blutpartikeln an die Stentoberfläche, das heißt an diese Beschichtung erschwert.

Wird ein mit einer solchen Beschichtung versehener Stent nun in dem Blutgefäß radial ausgedehnt, so werden derartige Beschichtungen ebenfalls mit gedehnt.

Dabei hat sich herausgestellt, daß diese Beschichtungen aus dem Stand der Technik nur in begrenztem Umfang in der Lage sind, der entsprechenden Dehnung des Stents zu folgen. Daher platzen diese Stentbeschichtungen von der Stentoberfläche ab, wenn die Dehnung der Stents und damit die in der Beschichtung verursachte Spannung zu groß wird.

EP 0 923 953 A2, EP 0 916 317 A1, EP 0 824 900 A2, EP 0 689 807 A2 und US 5,674,276 offenbaren Stents mit Beschichtungen, die ungleichmäßig ausgebildet sind.

Aufgabe der Erfindung ist es daher, die vorgenannten Nachteile zu vermeiden und einen Stent der eingangs genannten Art zur Verfügung zu stellen, dessen Beschichtung auch bei Dehnung des Stents nicht von dem Stent abplatzt.

Diese Aufgabe wird bei der Erfindung mit einem Stent der eingangs genannten Art dadurch gelöst, daß die Beschichtung ungleichmäßig auf der Stentoberfläche ausgebildet ist,
wobei die Beschichtung in Form von Beschichtungsinseln auf der Oberfläche des Stents ausgebildet ist. Der Stent zeichnet sich dadurch aus, dass
(i) die Beschichtungsinseln in einem unterschiedlich großen Abstand zueinander auf der Oberfläche angeordnet sind, wobei die Beschichtungsinseln in Bereichen größerer lokaler Dehnung der Stentoberfläche in einem größeren gegenseitigen Abstand angeordnet sind als in Bereichen kleinerer lokaler Dehnung der Stentoberfläche oder
(ii) die Beschichtungsinseln in Bereichen größerer lokaler Dehnung der Stentoberfläche kleiner sind als in Bereichen kleinerer lokaler Dehnung der Stentoberfläche

Die Beschichtungsinseln sind bevorzugt kreisrund ausgebildet. Denn derartige Beschichtungsinseln können in gleichmäßiger Form einfach aufgebracht werden und stellen gleichzeitig sicher, daß in der Beschichtungsinsel selber eine gleichmäßige Verteilung der Dehnungskräfte auf die Beschichtung erfolgt. Auf diese Weise werden Dehnungsspitzen in der Beschichtung selbst vermieden; so ist bei einer derartigen kreisrunden Beschichtungsinsel die Dehnbelastung der Beschichtungsinsel innerhalb der Beschichtungsinsel im wesentlichen gleichmäßiger als bei unregelmäßig geformten Inseln.

Die Erfndung kann auch verschiedenen Anforderungen des Stents angepaßt werden. So ist es je nach der Stentstruktur vorteilhaft, wenn alle Beschichtungsinseln im wesentlichen gleich groß sind. Bei dieser Ausführungsform ist es dann weiter bevorzugt, wenn die Beschichtungsinseln in Bereichen größerer lokaler Dehnung der Stentoberfläche bei Dehnung des Stents in einem größeren gegenseitigen Abstand angeordnet sind als in Bereichen kleinerer lokaler Dehnung der Stentoberfläche. Bei dieser Ausführungsform wird daher bei gleichgroßen Beschichtungsinseln durch unterschiedliche Beabstandung der Beschichtungsinseln auf der Stentoberfläche Rücksicht auf die unterschiedlichen Stärken der lokalen Dehnung des Stents genommen.

Bei anderen Stentstrukturen hat es sich als vorteilshaft erwiesen, wenn die gegenseitigen Abstände der Beschichtungsinseln im wesentlichen überall auf der Stentoberfläche gleich groß sind. Bei dieser Ausführungsform wird die Anpassung der Beschichtung an die unterschiedliche Dehnung des Stents dadurch vorgenommen, daß die Beschichtungsinseln in den Bereichen größerer lokaler Dehnung der Stentoberfläche kleiner sind als in Bereichen kleinerer lokaler Dehnung der Stentoberfläche. Bei dieser Ausführungsform wird die Anpassung der Beschichtung an unterschiedliche lokale Dehnung des Stents daher über die Größe der Beschichtungsinseln vorgenommen.

Bei einer weiteren besonders bevorzugten Ausführungsform der vorliegenden Erfindung ist die Beschichtung aus biokompartiblem Material ausgestaltet. Auf diese Weise läßt sich eine optimale medizinische Verträglichkeit des Stents erreichen.

Weitere bevorzugte Ausführungsformen der Erfindung sind in den Unteransprüchen angegeben.

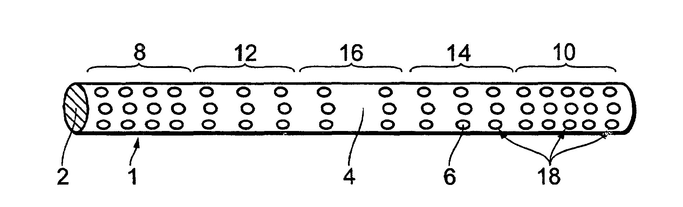

Ein Ausführungsbeispiel der vorliegenden Erfindung wird nun anhand der einzigen Figur beschrieben.

Die Figur zeigt einen Abschnitt 1 eines erfindungsgemäßen Stents. Der Abschnitt 1 besteht aus einem Stück Draht 2 der Struktur des Stents.

Der Draht 2 ist zylinderförmig ausgebildet und weist eine Oberfläche 4 auf. Auf der Oberfläche 4 befindet sich eine rasterförmig angeordnete Beschichtung 6.

Der Draht 2 ist derart in eine Stentstruktur eingebunden, daß es bei einer (nicht dargestellten) Dehnung des Stents zu unterschiedlichen lokalen Dehnungen des Drahtes 2 kommt. So werden die in der Figur dargestellten Bereiche 8 und 10 des Drahtes 2 nur gering gedehnt. Die Bereiche 12 und 14 des Drahtes 2 werden demgegenüber stärker gedehnt, wenn der Stent insgesamt gedehnt wird. Am stärksten wird der Bereich 16 des Drahtes 2 gedehnt, wenn der Stent gedehnt wird.

Die Beschichtung 6 ist auf der Oberfläche 4 des Drahtes 2 entsprechend der unterschiedlichen Dehnung der Bereiche 8 bis 16 in einer unterschiedlichen Dichte aufgebracht. Diese unterschiedliche Dichte wird dadurch erreicht, daß die einzelnen Beschichtungsinseln 18 der Beschichtung 6, die jeweils kreisförmig ausgebildet sind, in einem unterschiedlich großen Abstand zueinander auf der Oberfläche 4 angeordnet sind. So sind diese im wesentlichen gleich großen Beschichtungsinseln 18 in den Bereichen 8 und 10 in einem ersten Abstand zueinander angeordnet, während sie in den Bereichen 12 und 14 in einem zweiten, größeren Abstand zueinander angeordnet sind, und in dem Bereich in einem dritten, noch größeren Abstand zueinander angeordnet sind. Auf diese Weise kann die Beschichtung 6 die unterschiedlich starke Dehnung der Oberfläche 4 mitmachen, ohne daß es zu einem Abplatzen der Beschichtung 6 bzw. der Beschichtungsinseln 18 von der Oberfläche 4 des Drahtes 2 des Stents bei der Dehnung kommt.

## Patentansprüche

1. Stent, insbesondere Koronarstent, mit einer Beschichtung (6,18), die ungleichmäßig ausgebildet ist, wobei die Beschichtung (6, 18) in Form von Beschichtungsinseln (18) auf der Oberfläche (4) des Stents ausgebildet ist,
**dadurch gekennzeichnet, dass**
(i) die Beschichtungsinseln (18) in einem unterschiedlich großen Abstand zueinander auf der Oberfläche (4) angeordnet sind, wobei die Beschichtungsinseln (18) in Bereichen (12, 14, 16) größerer lokaler Dehnung der Stentoberfläche (4) in einem größeren gegenseitigen Abstand angeordnet sind als in Bereichen (8, 10) kleinerer lokaler Dehnung der Stentoberfläche (4) oder
(ii) die Beschichtungsinseln (18) in Bereichen (12, 14, 16) größerer lokaler Dehnung der Stentoberfläche (4) kleiner sind als in Bereichen (8, 10) kleinerer lokaler Dehnung der Stentoberfläche (4).

2. Stent nach Anspruch 1, wobei die Beschichtungsinseln (18) im wesentlichen kreisrund ausgebildet sind.

3. Stent nach Alternative (i) des Anspruchs 1, wobei die Beschichtungsinseln (18) im wesentlichen alle gleich groß sind.

4. Stent nach Alternative (ii) des Anspruchs 1, wobei die gegenseitigen Abstände der Beschichtungsinseln (18) im wesentlichen überall gleich sind.

5. Stent nach einem der vorstehenden Ansprüche, wobei die Beschichtung (6, 18) aus biokompatiblem Material besteht.

6. Stent nach einem der vorstehenden Ansprüche, wobei die Beschichtung amorphes Siliziumcarbid (a-SiC), hydrogenisiertes amorphes Siliziumcarbid. (a-SiC:H), und/oder Gold enthält.

## Claims

1. A stent, in particular a coronary stent, having a coating (6, 18), which is implemented unevenly, the coating (6, 18) being implemented in the form of coating islands (18) on the surface (4) of the stent,
**characterized in that**
(i) the coating islands (18) are positioned at different distances to one another on the surface (4), the coating islands (18) being positioned at greater mutual distances in areas (12, 14, 16) of greater local stretching of the stent surface (4) than in areas (8, 10) of lesser local stretching of the stent surface (4), or (ii) the coating islands (18) are smaller in areas (12, 14, 16) of greater local stretching of the stent surface (4) than in areas (8, 10) of lesser local stretching of the stent surface (4).

2. The stent according to Claim 1, wherein the coating islands (18) are implemented as essentially circular.

3. The stent according to alternative (i) of Claim 1, wherein the coating islands (18) are essentially all equally large.

4. The stent according to alternative (ii) of Claim 1, wherein the mutual distances of the coating islands (18) are essentially equal overall.

5. The stent according to one of the preceding claims, wherein the coating (6, 18) is made of biocompatible material.

6. The stent according to one of the preceding claims, wherein the coating contains amorphous silicon carbide (a-SiC), hydrogenated amorphous silicon carbide (a-SiC:H), and/or gold.

## Revendications

1. Stent, en particulier stent coronaire, avec un revêtement (6, 18), qui est conçu irrégulier, le revêtement (6, 18) étant réalisé sous la forme d'îlots de revêtement (18) sur la surface (4) du stent,
**caractérisé en ce que**
(i) les îlots de revêtement (18) sont disposés sur la surface (4) à une distance différente les uns des autres, les îlots de revêtement (18) étant disposés dans des zones (12, 14, 16) d'extension locale plus grande de la surface du stent (4) à une distance réciproque plus grande que dans des zones (8, 10) d'extension locale plus petite de la surface du stent (4) ou
(ii) les îlots de revêtement (18) dans des zones (12, 14, 16) d'extension locale plus grande de la surface du stent (4) étant plus petits que dans des zones (8, 10) d'extension locale plus petite de la surface du stent (4).

2. Stent selon la revendication 1, les îlots de revêtement étant réalisés sensiblement circulaires.

3. Stent selon l'alternative (i) de la revendication 1, les îlots de revêtement (18) étant sensiblement tous de même grandeur.

4. Stent selon l'alternative (ii) de la revendication 1, les espacements réciproques des îlots de revêtement (18) étant sensiblement partout identiques.

5. Stent selon l'une quelconque des revendications précédentes, le revêtement (6, 18) étant à base de matériau biocompatible.

6. Stent selon l'une quelconque des revendications précédentes, le revêtement contenant du carbure de silicium amorphe (a-SiC), du carbure de silicium amorphe hydrogéné (a-SiC:H), et/ou de l'or.
